# EUROPEAN PATENT APPLICATION

(11) **EP 3 789 028 A1**
(43) Date of publication of application: **10.03.2021**
(21) Application number: 19196185.3
(22) Date of filing: 09.09.2019
(51) Int. Cl.: A61K 31/515, A61P 9/00, A61P 17/00, A61P 19/00, A61P 19/02, A61P 25/00, A61P 37/00, A61P 43/00

(54) **PRIMIDONE IN THE USE FOR INHIBITING CELL DEATH IN DISEASES LIKE REPERFUSION INJURY DISEASE, NEURODEGENERATIVE DISEASE ETC**

(71) Applicant: Christian-Albrechts-Universität zu Kiel, 24118 Kiel (DE)
(72) Inventor: KRAUTWALD, Stefan, 24220 Flintbek (DE); KUNZENDORF, Ulrich, 24105 Kiel (DE)
(74) Representative: Uexküll & Stolberg

(57) **Abstract**

The present invention relates to the field of diseases or conditions that involve a pathologic level of RIPK1-dependent cell death. Specifically, the present invention refers to the use of the compound primidone or a pharmaceutically acceptable active metabolite, derivative, salt or solvate thereof for treating a disease or condition that involves a pathologic degree of RIPK1-dependent cell death. In a further aspect, the present invention provides a pharmaceutical composition comprising primidone or a pharmaceutically acceptable active metabolite, derivative, salt or solvate thereof for treating a disease or condition that involves a pathologic degree of RIPK1-dependent cell death.

## Description

The present invention relates to the field of diseases or conditions that involve a pathologic level of RIPK1-dependent cell death. Specifically, the present invention refers to the use of the compound primidone or a pharmaceutically acceptable active metabolite, derivative, salt or solvate thereof for treating a disease or condition that involves a pathologic degree of RIPK1-dependent cell death. In a further aspect, the present invention provides a pharmaceutical composition comprising primidone or a pharmaceutically acceptable active metabolite, derivative, salt or solvate thereof for treating a disease or condition that involves a pathologic degree of RIPK1-dependent cell death.

### BACKGROUND OF THE INVENTION

Necroptosis, a regulated form of necrosis, is a caspase-independent programmed cell death mechanism. Necroptosis is mediated by receptor-interacting protein kinase 3 (RIPK3) activation and the pursuant RIPK3-mediated phosphorylation of its pseudokinase substrate mixed lineage kinase domain-like protein (MLKL) (Cho et al., 2009; Sun et al., 2012). This initial stimulus prompts a conformational change that results in MLKL oligomerization, plasma membrane translocation, and lethal permeation of the lipid bilayer, leading to the release of cellular content, which triggers an inflammatory response.

The signaling pathways of various subtypes of regulated cell death have been analysed in detail in recent years. Another protein, receptor-interacting protein kinase 1 (RIPK1), appears to play a crucial role in the activation of regulated cell death, and it has been suggested that the inhibition of this protein might offer therapeutic benefit in a number of diseases which are characterized by a pathogenic level of apoptosis and necroptosis, such as ischemia-reperfusion injuries like myocardial infarction and acute kidney injury, neurodegenerative disorders like Parkinson's and Alzheimer's disease as well as stroke, sepsis and cancer. Although RIPK1 is currently considered as an important drug target by the pharmaceutical industry, there is so far no approved therapeutic drug that would allow inhibiting RIPK1-dependent apoptosis and necroptosis, thereby reversing or ameliorating the symptoms of the aforementioned diseases.

Against this background, it is an objective of the present invention to provide pharmaceutically active compounds and compositions that effectively inhibit RIPK1-mediated cell death and allow for the treatment of a disease or condition that involves a pathologic level of cell death. According to the invention, this objective is achieved by the compounds and pharmaceutical compositions defined in the enclosed claims.

The present invention is based on the surprising insight that the well-known anticonvulsant compound primidone is able to block RIPK1-dependent apoptosis and necroptosis by inhibiting RIPK1 activation. Accordingly, primidone and its active metabolites, derivatives, salts and solvates will be highly useful for blocking cell death in diseases or conditions which are associated with a pathological level or activation of RIPK1-dependent cell death.

### DESCRIPTION OF THE INVENTION

Activating necroptosis is known to require the activity of receptor-interacting protein 1 (RIPK1), a protein which mediates the activation of the two proteins RIPK3 and MLKL, both of which are critical downstream mediators of necroptosis. Owing to its central function in the necroptosis pathway, RIPK1 inhibitors have been suggested as useful therapeutics that are suitable to prevent the pathologic level of cell death which occurs in a number of diseases.

The present invention contemplates the use of the compound primidone for treating a disease or condition that involves a pathologic level or activation of RIPK1-dependent cell death. It was found in the course of this invention that primidone effectively inhibits RIPK1 activity in cells and tissues. As such, primidone is suitable for preventing or inhibiting RIPK1-dependent cell death, in particular apoptosis and necroptosis, in numerous diseases and conditions, including ischemia-reperfusion injury, neurodegenerative diseases like Parkinson's or Alzheimer's disease and sepsis.

Primidone is an anticonvulsant of the barbiturate class that is approved for a long time for therapeutic treatment of seizures, including partial and generalized seizures, and tremors. It is sold, amongst others, under the trade names Lepsiral®, Mysoline®, Resimatil®, and Liskantin®. Primidone is also known as desoxyphenobarbital or desoxyphenobarbitone. The IUPAC name of primidone is 5-Ethyl-5-phenyl-1,3-diazinane-4,6-dione. It was found herein that primidone prevents the activation of RIPK1 that normally occurs by (auto)phosphorylation. In the absence of phosphorylated RIPK1, the assembly of the necrosome consisting of the proteins RIPK1, RIPK3 and MLKL cannot occur. This ultimately leads to the prevention of RIPK1-mediated cell death.

In a first aspect, the present invention relates to the compound primidone or a pharmaceutically acceptable active metabolite, derivative, salt or solvate thereof, or a pharmaceutical composition comprising any of those, for use in a method of treating a disease or condition that involves a pathologic level of RIPK1-dependent cell death.

In one preferred embodiment, the disease to be treated is selected from the group consisting of reperfusion a injury disease, a systemic inflammatory disease, a transplantation-related disease, a neurodegenerative disease, an autoimmune disease, an ophthalmologic disease, and a RIPK-expressing tumor disease.

In one embodiment, the disease or condition to be treated that involves a pathologic level of RIPK1-dependent cell death is a reperfusion injury disease. As used herein, a "reperfusion injury disease" includes diseases and conditions that involve tissue damage caused by reperfusion, i.e. tissue damage that occurs when the blood supply to tissue is restored after a period of ischemia or lack of oxygen. Tissue damage occurring after reperfusion is known to involve RIPK1-dependent processes. The importance of RIPK1-dependent cell death following ischemia and reperfusion has been demonstrated in rodent models (Linkermann et al. (2012)). Accordingly, it has been suggested that blocking RIPK1 activity could be a viable strategy for reducing ischemia reperfusion-related cell death in reperfusion injuries of the brain, retina, heart, kidney, and liver.

In one embodiment, the reperfusion injury disease to be treated is stroke. One of the main events occurring during ischemic brain stroke is cell death (Liu C. et al. (2017)). It is well-known that necroptosis mediated by RIPK and MLKL proteins contributes to brain injury after ischemic stroke. Therefore, primidone can effectively protect brain tissue from ischemic injury by blocking RIPK1-dependent necroptosis.

In another embodiment, the reperfusion injury disease to be treated is myocardial infarction (heart attack). Regulated necrosis is one of the main forms of cardiomyocyte death in heart disease and heart failure. Especially RIPK1-mediated necroptosis has been implicated in the progression of myocardial infarction (Oerlemans MI, et al. (2012)). Therefore, RIPK1 might serve as a novel therapeutic target for prevention of myocardial ischemia-reperfusion injury.

In yet another embodiment, the reperfusion injury disease to be treated is acute kidney failure Linkermann et al. (2012) or acute liver failure (Takemoto K. et al (2014)).

In another embodiment, the disease or condition to be treated that involves a pathologic level of RIPK1-dependent cell death is a systemic inflammatory disease which is preferably selected from the group consisting of sepsis and systemic inflammatory response syndrome (SIRS). Cell death mechanisms play an important role in the pathogenesis of these diseases. For this reason, the use of RIPK1 inhibitors for blocking RIPK1-dependent cell death has been considered particularly useful for treating sepsis (Degterev A. et al. (2019); Zelic M. (2018)).

In yet another embodiment, the disease or condition to be treated that involves a pathologic level of RIPK1-dependent cell death is a transplantation-related disease, such as graft-versus-host-disease. Cell death processes like apoptosis and necroptosis were found to contribute to organ failure during organ transplantation and in graft-versus-host-disease (Shi, S. et al. (2019), Falcon, C. et al. (2017); Kanou T. et al. (2018); Pavlosky A (2014)).

In another preferred embodiment, the disease to be treated that involves a pathologic level of RIPK1-dependent cell death is a neurodegenerative disease selected from Alzheimer's disease, Parkinson's disease, Huntington's disease, amyotrophic lateral sclerosis (ALS), traumatic brain injury, and multiple sclerosis (MS). It is well known that RIPK1-dependent necroptosis promotes cell death and neuroinflammation in the pathogenesis of these diseases (Yuan, J. et al. (2019)).

In yet another embodiment, the disease to be treated that involves a pathologic level of RIPK1-dependent cell death is an autoimmune disease. RIPK1-dependent cell death was found to be involved in the pathomechanisms that result in autoimmune disease like ulcerative colitis, Crohn's disease, rheumatoid arthritis, autoimmune cardiomyopathy, autoimmune hepatitis, lupus erythematosus, Graves' disease, Guillain-Barre syndrome (GBS), Hashimoto's thyroiditis, idiopathic thrombocytopenic purpura, juvenile idiopathic arthritis, myasthenia gravis, pemphigus vulgaris, psoriasis, Reiter's syndrome, scleroderma, Sjögren's syndrome, vasculitis, vitiligo, and Wegener's granulomatosis (Degterev A. et al. (2019)).

In yet another embodiment, the disease to be treated is an ophthalmologic disease, such as retinal degeneration or retinitis pigmentosa (Sato K. et al. (2013); Do YJ et al. (2017)).

In yet another embodiment, the disease to be treated is a cancer disease resulting from a tumor that overexpresses RIPK1. For example, an upregulation of RIPK1 has been described in lung and pancreatic tumours (Gong Y. et al. (2019)). Thus, in a preferred aspect of the invention, the disease to be treated is a cancer disease resulting from a RIPK1-expressing lung or pancreatic tumor.

As used herein, primidone refers to the compound 5-Ethyl-5-phenyl-1,3-diazinane-4,6-dione having the following structural formula:

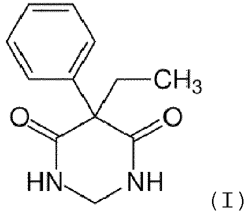

Also included according to the invention are derivatives of the above structure (I) which are substituted or otherwise modified at one or several positions, as long as these modifications neither substantially affect the inhibitory effect of primidone on the RIPK1 activity nor lead to adverse results in terms of toxicity. For example, one or more hydrogen atoms of the C-H bonds of the heterocyclic ring systems can be substituted by halogen atoms, such as chlorine, bromine or iodine atoms. Further, the hydrogen of the C-H bonds can also be replaced by a short-chained alkyl group such as methyl, ethyl, propyl, or a long-chained alkyl group.

Active metabolites of primidone are also included by the invention. In particular, the invention also relates to the use of the primidone metabolite phenobarbital (PB), also known as phenobarbitone or phenobarb, for blocking RIPK1-dependent cell death. As used herein, phenobarbital refers to the compound 5-Ethyl-5-phenyl-1,3-diazinane-2,4,6-trione having the following structural formula:

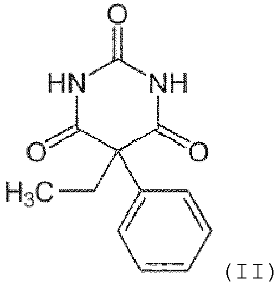

The invention also refers to the use of the primidone metabolite phenylethylmalonamide (PEMA), for blocking RIPK1-dependent cell death. As used herein, PEMA refers to the compound 2-Ethyl-2-phenylmalonamide having the following structural formula:

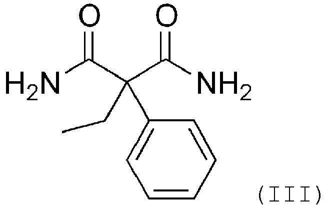

Also included according to the invention are derivatives of the above-depicted metabolite structures (II) and (III) which are substituted or otherwise modified at one or several positions, as long as these modifications neither substantially affect the inhibitory effect of the primidone metabolite on the RIPK1 activity nor lead to adverse results in terms of toxicity. For example, one or more hydrogen atoms of the C-H bonds of the heterocyclic ring systems can be substituted by halogen atoms, such as chlorine, bromine or iodine atoms. Further, the hydrogen of the C-H bonds can also be replaced by a short-chained alkyl group such as methyl, ethyl, propyl, or a long-chained alkyl group.

Pharmaceutically acceptable salts of primidone or its active metabolites or derivatives can also be used for treating the above diseases via RIPK1 inhibition. The term "pharmaceutically acceptable salt" refers to non-toxic acid addition salts, and alkali metal and alkaline earth metal salts, respectively. Exemplary acid addition salts include hydrochloride, hydrobromide, sulfate, bisulfate, acetate, oxalate, phosphate, citrate, maleate, fumarate, succinate, tartrate and lauryl sulfate. Exemplary alkali metal or alkaline earth metal salts include sodium, potassium, calcium and magnesium salts. In addition, ammonium salts and salts with organic amines can be used as well. Apart from the calcium salt, any other pharmaceutically acceptable cationic salt can be employed. The salts are, for example, salts that are obtained by reaction of the free acid form of primidone with a suitable base, such as sodium hydroxide, sodium methoxide, sodium hydride, potassium methoxide, magnesium hydroxide, calcium hydroxide, choline, diethanolamine, and others which are known in the prior art. Solvates of primidone or its metabolites are also part of the present invention. Solvates occur upon the addition of one or more solvent molecules to primidone or its metabolites. If the solvent is water, said addition is a hydration. Solvates of the contemplated active ingredient compound can be held together by ionic bonds and/or covalent bonds.

Primidone or the active metabolite, derivative, salt or solvate thereof will be formulated as a pharmaceutical composition suitable for administration to a subject, preferably a human subject. Pharmaceutical compositions comprising primidone can be formulated in accordance with standard methods using commonly known excipients. Such methods and suitable excipients and carriers are described, for example, in "Remington: The Science and Practice of Pharmacy", Lippincott Williams & Wilkins; 21st ed. (2005). Pharmaceutical compositions comprising primidone or an active metabolite, derivative, salt or solvate thereof may be formulated, for example, as compositions for oral, rectal, nasal or parental (including subcutaneous, intramuscular, intravenous and intradermal) administration. Depending on the target tissue to be treated, the compositions can occur in the form of granules, powders, tablets, capsules, syrup, suppositories, injection solutions, emulsions or suspensions.

Normally, the pharmaceutical compositions of the invention comprise primidone or an active metabolite, derivative, salt or solvate thereof in admixture with one or more pharmaceutically acceptable carriers which are physiologically compatible with the other ingredients in the compositions. The pharmaceutical compositions of the invention can also include further excipients, such as binders, diluents, dyes, sweeteners or the like.

Pharmaceutical compositions for oral, buccal, or sublingual administration may be provided as solid formulations, such as powders, suspensions, granules, tablets, pills, capsules or gel caps. Suitable carriers that are often used in solid compositions are discussed in "Remington: The Science and Practice of Pharmacy", Lippincott Williams & Wilkins; 21st ed. (2005) and comprise, for example, microcrystalline cellulose, methylcellulose, hydroxypropyl methylcellulose, casein, albumin, mannitol, dextran, sucrose, lactose, sorbitol, starch, agar, alginate, pectins, collagen, glycerides, or gelatine. Solid compositions for oral, buccal, or sublingual administration are may also comprise antioxidants, such as ascorbic acid, tocopherol or cysteine, lubricants, such as magnesium stearate, preservatives, such as paraben or sorbic acid, disintegrants, binders, thickeners, taste enhancers, dyes and the like.

Alternatively, pharmaceutical compositions for oral, buccal, or sublingual administration may also be provided as liquid formulations, for example, as emulsions, syrups, suspensions or solutions. These formulations can be prepared by admixing primidone or its active metabolite, derivative, salt or solvate with a liquid carrier which may be a sterile liquid, such as oil, water, alcohol or combinations thereof. Oils which are suitable for being used in liquid dosage forms comprise, for example, olive oil, sesame oil, peanut oil, rape oil, and corn oil. Suitable alcohols comprise ethanol, isopropyl alcohol, hexadecyl alcohol, glycerol and propylene glycol. If the pharmaceutical composition is formulated in the form of a suspension, a fatty acid ester, such as ethyl oleate or isopropyl myristate, a fatty acid glyceride, or an acetylated fatty acid glyceride may be added. Furthermore, substances like mineral oil or petrolatum are often added to suspensions.

It is preferred herein that the pharmaceutical composition of the invention is formulated for being administered by injection, e.g. by bolus injection or by continuous infusion. Compositions suitable for injection typically comprise an aqueous solution, an aqueous suspension or an oil suspension which has been prepared by use of a suitable solvent or suspending agent. Injectable compositions may also comprise stabilizers or surfactants that can optionally be added to these formulations. Injectable compositions may be formulated in the form of powders that can be reconstituted in a suitable solvent prior to use. Examples include, amongst others, lyophilized or spray-dried powders.

The route of administration which is most suitable for the respective therapy and the amount of the primidone compound to be administered can be determined by a skilled person using routine methods. Parameters which influence the amount of primidone or an active metabolite, derivative, salt or solvate thereof in the pharmaceutical composition to be administered include the type and severity of the disease, age, body weight, sex and overall state of health of the patient to be treated, the simultaneous administration of other therapeutic agents, and other parameters.

Suitable pharmaceutical compositions for the administration to humans will typically comprise 1 mg to 50 mg of the primidone compound per kilogram body weight of the patient. For children, primidone is typically administered at 1 mg to 30 mg of the primidone compound per kilogram body weight, preferably at 5 mg to 20 mg of primidone per kilogram body weight, and more preferably at 10 mg to 20 mg of primidone per kilogram body weight. For adults, primidone is typically administered at 1 mg to 20 mg of the primidone compound per kilogram body weight, preferably at 5 mg to 15 mg of the primidone compound per kilogram body weight, and more preferably at 10 mg to 15 mg of the primidone compound per kilogram body weight. Stated differently, the overall amount of the primidone compound administered to the patient daily typically is in the range from 5 mg to 5000 mg, preferably from 50 mg to 2500 mg, and more preferably from 500 mg to 1500 mg.

It is preferred that primidone or the active metabolite, derivative, salt or solvate thereof is administered in an amount that the concentration of the primidone compoin the plasma is between 0.1 µg/ml and 12 µg/ml, preferably between 0.5 µg/ml and 10 µg/ml, more preferably between 1 µg/ml and 7.5 µg/ml, and even more preferably between 2 µg/ml and 5 µg/ml. The therapy can be monitored and adapted accordingly to provide for such plasma concentrations.

The therapeutic effectiveness of the pharmaceutical compositions of the invention can be evaluated by using parameters known in the art. These parameters comprise, amongst others, the effectiveness of the composition according to the invention in eradicating symptoms of the treated disease, the response rate, the time until progression of the disease and the survival rate of the treated patients. Preferably, the compositions of the invention lead to a complete response in the patient. As used herein, complete response means the elimination of all clinically detectable disease symptoms as well as the restoration of normal results in blood count, x-ray examination, CT pictures, and the like. Such a response lasts preferably a month after the treatment has been stopped. The anti-compositions of the invention can also result in a partial response in the patient. In a partial response, the measurable tumor burden in the patient is reduced. At the same time, an improvement occurs in one or more symptoms which are caused by the disease, e.g. fever, loss of weight, and the like. Primidone or the active metabolite, derivative, salt or solvate thereof can be used in combination with at least one additional therapeutic agent, such as another inhibitor of apoptosis or necroptosis, like Nec-1 or Nec1s. The primidone compound and the additional therapeutic agent can be administered to the subject in the form of a single pharmaceutical composition comprising both compounds agents, optionally in combination with suitable excipients and carriers. Administration of such a pharmaceutical composition will then automatically result in a simultaneous administration of the primidone compound and the additional therapeutic agent to the subject. Alternatively, the primidone compound and the additional therapeutic agent may also be administered separately from each other, i.e. in the form of two separate pharmaceutical compositions, one containing the primidone compound, and the other containing the additional therapeutic agent. The two separate compositions can be administered simultaneously or sequentially in either order to the same or different administration sites.

For example, a combination therapy with the above-mentioned agents may begin at day 1 of a treatment period, and a first therapeutically effective dose of the primidone compound is administered at that day. A first therapeutically effective dose of the other therapeutic agent, such as another inhibitor of apoptosis or necroptosis, like Nec-1 or Nec1s, can be administered either on the same day, e.g. simultaneously or within about 30, 60, 90, 120, 150, 180, 210 or 240 minutes after administration of the primidone compound. Alternatively, the other therapeutic agent can be administered 2, 3, 4, 5, 6 or 7 days after administration of the primidone compound. A skilled person will readily be able to design administration regimens which are suitable for providing the maximum therapeutic effect.

### BRIEF DESCRIPTION OF THE FIGURES

Fig. 1 shows the results of stimulation assays demonstrating that primidone (referred to as compound A) blocks both RIPK1-dependent apoptosis and RIPK1-dependent necroptosis.
Fig. 2 shows the results of stimulation assays demonstrating that primidone not only blocks RIPK1-dependent cell death which is induced by ligation of death receptors like TNFR1 but also by endosomal Toll-like receptor 3 (TLR3) agonist which serves as a sensor of viral infection and sterile tissue necrosis.
Fig. 3 shows the results of stimulation assays demonstrating that primidone (referred to as compound A) does not repress NF-κB activation.
Fig. 4 shows the results of profiling kinase assay demonstrating that primidone (referred to as compound A), unlike Nec-1s, does not inhibit the kinase activity of RIPK1.
Fig. 5 shows the results of binding studies demonstrating that during TSZ-induced necroptosis RIPK1 binds to the TNF-α receptor in the presence of primidone.
Fig. 6 shows the results of stimulation assays demonstrating that during TSZ-induced necroptosis primidone prevents the phosphorylation (activation) of RIPK1, thereby inhibiting the assembly of the necrosome.
Fig. 7 shows the results of stimulation assays demonstrating that primidone specifically blocks RIPK1-mediated cell death.
Fig. 8 shows the results of stimulation assays demonstrating that primidone is longer active compared to Nec-1s.
Fig. 9 shows the results of stimulation assays demonstrating that both primidone metabolites, phenylethylmalonamide (PEMA) and phenobarbital (PB), respectively, block RIPK1-dependent cell death processes.

### EXAMPLES

The present invention will be described in the following by preferred embodiments which merely illustrate the invention, but should by no means limit the invention.

### Example 1: Primidone blocks RIPK1-mediated apoptosis and necroptosis

Murine fibroblasts (L929 cells) were stimulated for 24 h at 37°C in the presence of (a) vehicle, (b) 10 ng/ml tumor necrosis factor alpha (TNF-α), (c) 1 µM 5Z-7-oxozeaenol (5Z-7), (d) a combination of 10 ng/ml TNF-α and 1 µM 5Z-7, (e) a combination of 10 ng/ml TNF-α, 1 µM 5Z-7, and 20 µM necrostatin-1 (Nec-1s), (f) a combination of 10 ng/ml TNF-α, 1 µM 5Z-7, and 1 mM primidone (primidone is referred to in the Figures as compound A), (g) a combination of 10 ng/ml TNF-α, 1 µM 5Z-7, and 25 µM zVAD, (h) a combination of 10 ng/ml TNF-α, 1 µM 5Z-7, and 20 µM Nec-1s, and (i) a combination of 10 ng/ml TNF-α, 1 µM 5Z-7, 25 µM zVAD, and 1 mM primidone. TNF-α is a pro-inflammatory cytokine which triggers cell survival (canonical pathway) or cell death, depending on the cellular context. 5Z-7-oxozeaenol (5Z-7) is an inhibitor of transforming growth factor activated kinase 1 (TAK1). TAK1 is an intermediate in the signaling pathway of the TNF-receptor type 1 (TNFR1), and it is known that TAK1 is essential for the prevention of TNF-induced cell death. Cells in which TAK1 is disrupted or otherwise blocked are hypersensitive to TNF-α-induced cell death due to diminished pro-survival pathways including NF-κB and reduced antioxidant enzymes which results in activation of caspases. The compound zVAD is a pan-caspase inhibitor. Nec-1s is a Necrostatin-1 analogue with superior selectivity and stability which can be purchased, e.g. from Abcam (Berlin, Germany), and inhibits the kinase activity of RIPK1.

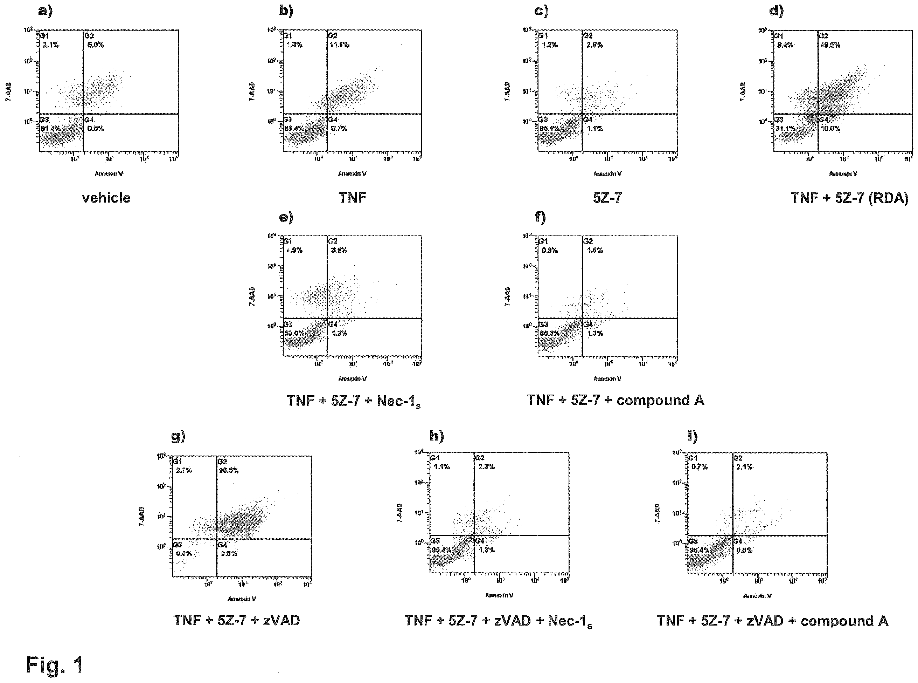

Results: As shown in panel (d) of Figure 1, the combination of TNF-α and 5Z-7 induces RIPK1-dependent apoptosis (RDA). As this cell death is RIPK1-dependent, it can be blocked with the RIPK1 inhibitor Nec-1s, as depicted in panel (e). As shown in panel (f), RIPK1-dependent apoptosis could also be blocked with primidone. Since the cell death induced by TNF-α + 5Z-7 is apoptotic, it should be possible to block it by adding a pan-caspase inhibitor like zVAD. It can be seen in panel (g) that cell death occurs, even though apoptosis is blocked which means that cell death shifts after inhibition of caspase-8 from apoptosis to necroptosis. This fact is confirmed in panel (h) showing that the addition of Nec-1s prevents RIPK-1 dependent cell death. Again, it was found that primidone was also able to block this RIPK-1-mediated necroptosis, see panel (i). In summary, this experiment shows that primidone can block both RIPK1-dependent apoptosis and RIPK1-dependent necroptosis.

### Example 2: Primidone blocks TLR3-mediated cell death

Cell death was induced by activation of the Toll-like receptor 3 (TLR3). TLR3 is mainly expressed on immune cells, where it senses pathogen-associated molecular patterns and initiates innate immune response. The TLR3 agonist poly (I:C) was developed to mimic pathogens infection and boost immune system activation. In experimental models, poly (I:C) is known to induce regulated cell death in cells expressing TLR3. To examine whether primidone also blocks TLR3-mediated cell death, murine L929 cells were stimulated for 24 h at 37°C with vehicle (a), 1 µg/ml of the TLR3 ligand poly (I:C) alone (b) or in combination with 25 µM zVAD (c). In addition, cells were stimulated with 1 µg/ml poly (I:C), 25 µM zVAD and 20 µM Nec-1s (d) and with 1 µg/ml poly (I:C), 25 µM zVAD and 1 mM primidone (e).

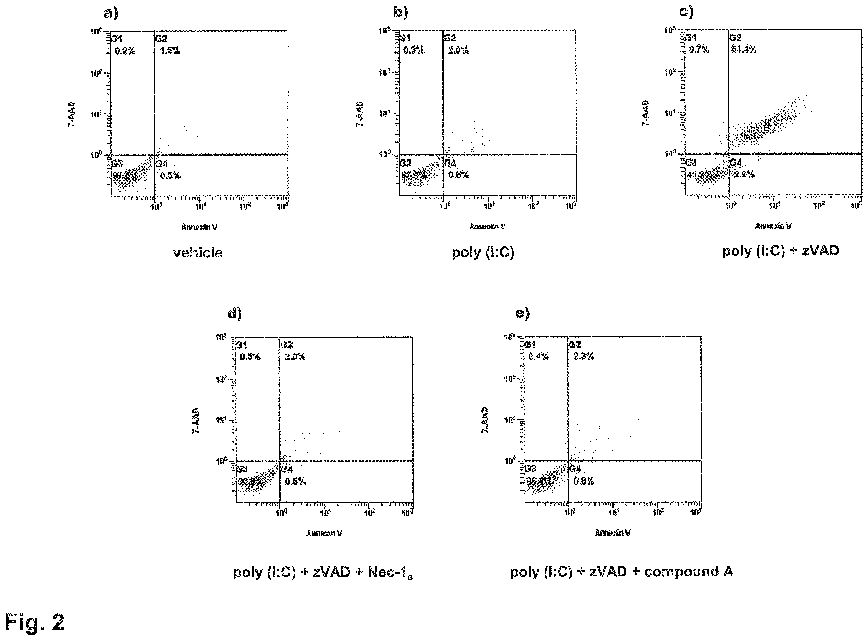

Results: As shown in panel (b) of Figure 2, the addition of poly (I:C) alone does not induce cell death. Cell fate decisions following TLR signaling parallel death receptor signaling and rely on caspase-8 to suppress RIPK-dependent programmed necrosis. Therefore, the combined administration of poly (I:C) and zVAD induces a cell death, as shown in panel (c). Both Nec-1s and primidone effectively protect cells from this RIPK1-mediated cell death induced by poly (I:C) and zVAD, as shown in panels (d) and (e), respectively.

### Example 3: Primidone does not repress the NF-κB pathway

It was then analyzed whether primidone interferes with the canonical NF-κB signaling pathway which drives the expression of pro-survival molecules. The binding of TNF-α to its corresponding receptor (TNFR1) initially results in the activation of the NF-κB signaling pathway. In this early phase of TNF activation, the cells are protected from cell death by the presence of a membrane-bound complex known as complex I which is formed within seconds after engagement of TNFR1 by TNF-α. This complex induces the expression of pro-survival molecules via activation of the canonical NF-κB pathway. As the default response of most cells to TNF-α is survival and NF-KB-mediated upregulation of pro-survival genes, it was analyzed whether primidone functions in a survival-signaling mode and thus indirectly prevents cell death signaling. For this purpose, murine L929 cells were stimulated at 37°C for different time periods (as indicated) with 100 ng/ml TNF-α + 25 µM zVAD (TZ) in the absence (vehicle) or presence of 1 mM primidone. Subsequently, a Western blot analysis of the cell lysates using a specific p-NF-κB antibody was performed.

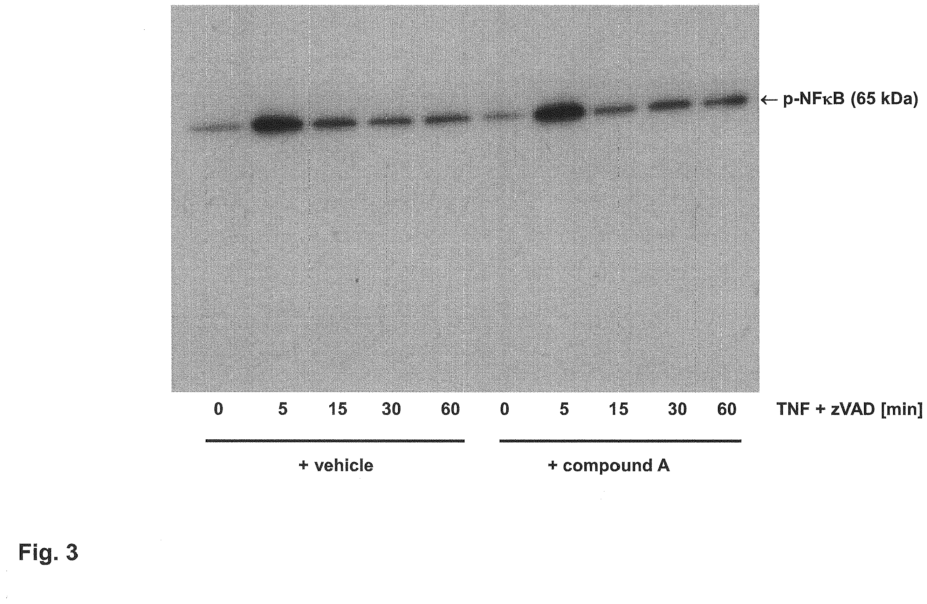

Results: Activation (phosphorylation) of NF-κB was detected 5 minutes after the treatment of the cells with TZ in the absence (vehicle) and in the presence of primidone (see Figure 3), indicating that primidone does not inhibit or degrade any of the proteins required for the formation of complex I which would then prevent the formation of the cell death-inducing complex II.

### Example 4: Primidone does not bind to the RIPK1 kinase domain

The kinase domain of RIPK1 is considered to have a key function in the signaling pathway. Known inhibitors of necroptosis like Nec-1s bind to the kinase domain of RIPK1, thereby interfering with the function of the protein. It was therefore investigated whether primidone binds to the kinase domain of RIPK1. To clarify, a KINOMEscan™ profiling kinase assay was performed. This assay measures the ability of compounds that bind to the kinase active site of RIPK1 to directly (i.e. sterically) or indirectly (i.e. allosterically) prevent kinase binding to an immobilized ligand. Dissociation constants (Kd) for test compound-kinase interactions are calculated by measuring the amount of kinase captured on the solid support as a function of the test compound concentration.

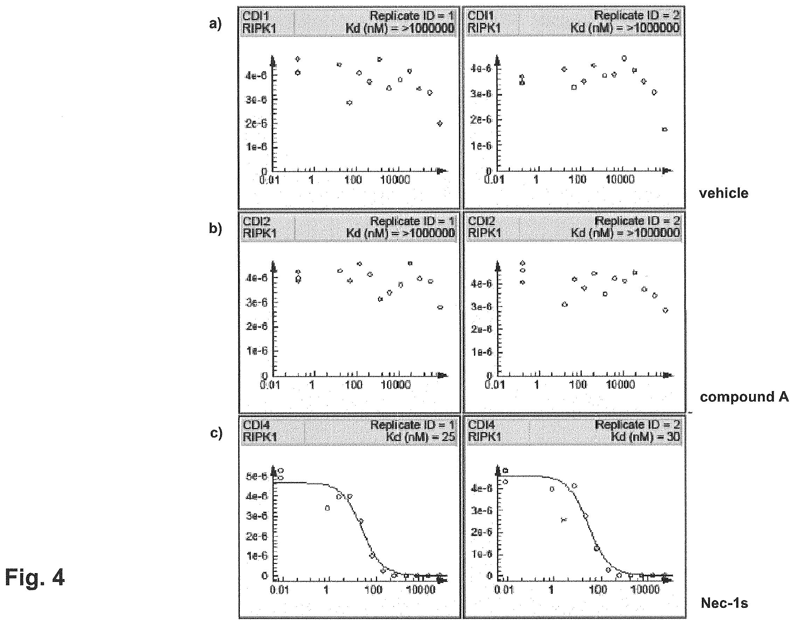

Results: It can be seen that Nec-1s, a compound that is known to bind to the kinase domain of RIPK1, prevents the kinase from binding to an immobilized ligand, as shown in row (c) of Figure 4. It can be taken from the panels that the dissociation constant (Kd) value plotted on the ordinate decreases constantly with increasing Nec-1s concentration. Such a course cannot be observed for rows (a) and (b) which reflect the results from the kinase assay obtained with vehicle (a) or primidone (b), respectively. This means that primidone, unlike Nec-1s, does not inhibit RIPK1 by binding directly to its kinase domain.

### Example 5: Primidone does not prevent complex I assembly

It was tested whether primidone interferes with binding of TNF-α to its corresponding receptor TNFR1. To induce cell death, human U937 cells were treated in the absence (vehicle) or presence of 1 mM primidone with 100 ng/ml Fc-tagged TNF-α + 1 µM SMAC mimetic SM164 + 25 µM zVAD (TSZ) for different time periods, followed by immunopurification of the immunoprecipitated TNF-α-induced complex I using µMACS™ protein A/G microbeads, and western blot analysis using a specific RIPK1 antibody.

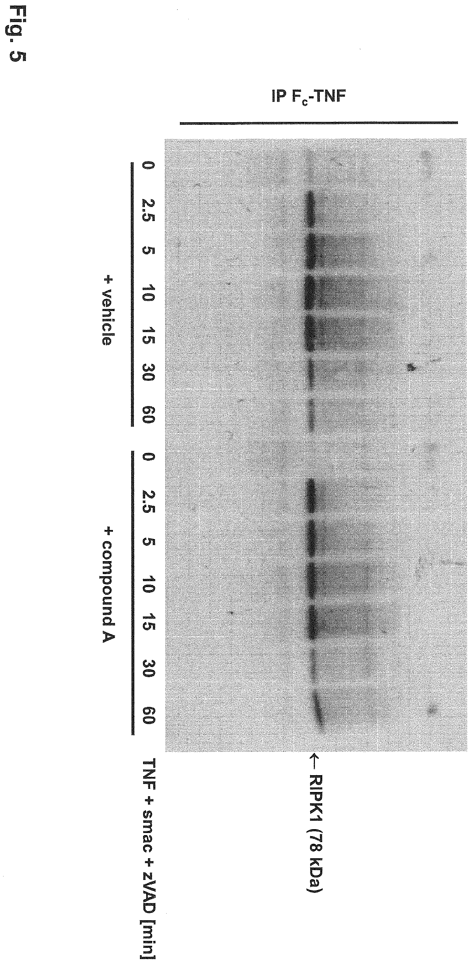

Results: As shown in Figure 5, RIPK1 binds to TNFR1 regardless of whether primidone was present during stimulation or not. The right half of Figure 5 shows the result in the presence of primidone, while the left half of Figure 5 shows the result without primidone. This indicates that the TNF-α-induced formation of complex I is not influenced at this stage by the presence of primidone.

### Example 6: Primidone prevents RIPK1 activation

It was then analyzed if the previously immunoprecipitated RIPK1 is activated by phosphorylation. For this purpose, the Western Blot shown before (Figure 5) was "stripped" by chemically removing all reagents from the blot and re-developed with an antibody that only detects RIPK1 in its activate (i.e. phosphorylated) form.

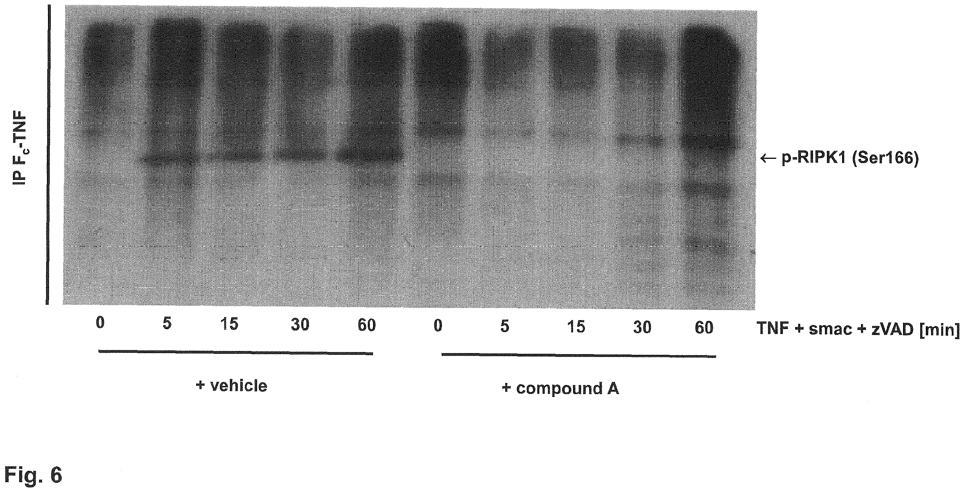

Results: It was found that TSZ treatment of the U937 cells resulted in a time-dependent activation (phosphorylation) of RIPK1 at residue Ser166 (left half of Figure 6), which is completely blocked by the addition of primidone (right half of Figure 6), indicating that primidone prevents the activation of RIPK1 which is a major function of RIP1 kinase in TNF-α-induced cell death.

### Example 7: Primidone blocks RIPK1-mediated cell death

Since primidone, amongst others, suppresses death receptor-initiated RIPK1-depependent signaling, it was tested whether primidone also inhibits death receptor-initiated processes that are not dependent on RIPK1. To this end, human T cells (Jurkat cells) were incubated for 5 h at 37°C in the presence of (a) vehicle, (b) 5 ng/ml anti-Fas antibody, (c) 5 ng/ml anti-Fas antibody in combination with 25 µM of the pan-caspase inhibitor zVAD, and (d) 5 ng/ml anti-Fas antibody in combination with 1 mM primidone. The addition of the anti-Fas antibody induces apoptosis, a caspase-dependent form of regulated cell death that can be inhibited by the addition of the pan-caspase inhibitor zVAD.

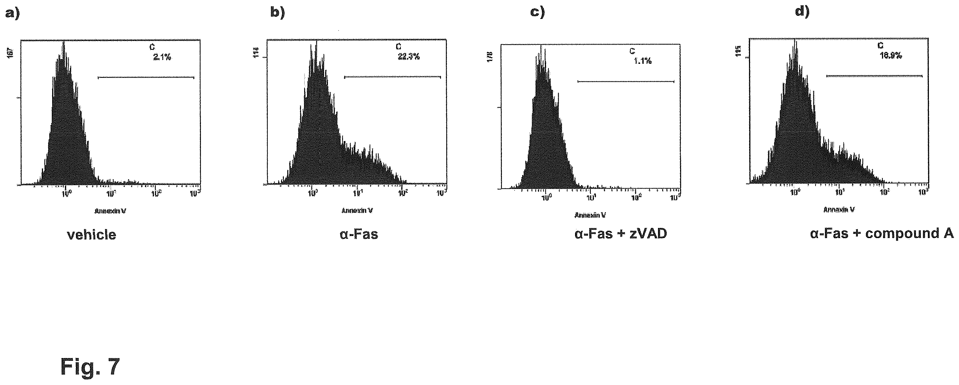

Results: The results are depicted in Figure 7. Panel (a) expectedly shows no cell death in the presence of the negative control. Panel (b) demonstrates that the addition of an anti-Fas antibody induces cell death. Cell death induced in this way is caspase-dependent, but RIPK-1-independent. Accordingly, cell death can be completely blocked by the addition of the pan-caspase inhibitor zVAD, see panel (c). By contrast, the addition of primidone has no inhibitory effect as shown in panel (d). It follows from this experiment that primidone specifically blocks RIPK1-mediated cell death.

### Example 8: Primidone shows longer activity than Nec-1s

The time profile of primidone for blocking cell death was compared to the one of Nec-1s. For this purpose, murine L929 cells were stimulated at 37°C for 24 h with 10 ng/ml TNF-α + 25 µM zVAD (TZ) in the presence of 1 mM primidone and 25 µM Nec-1s, respectively, for the indicated durations. Nec-1s and primidone were added 30 min before, 60 min and 180 min after the induction of RIPK1-mediated necroptosis, respectively. Cell death was quantified by FACS analysis using 7-amino-actinomycin D and phosphatidylserine accessibility (Annexin V staining) as markers.

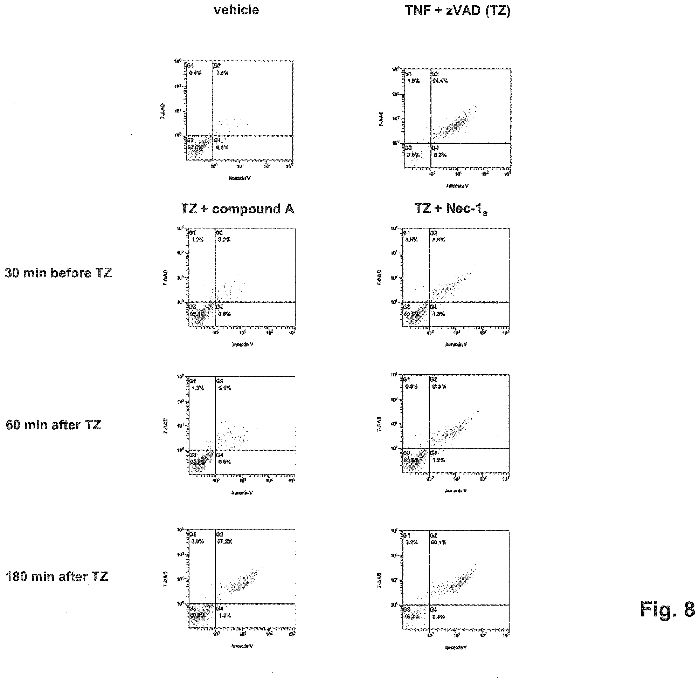

Results: The results are depicted in Figure 8. Data of one representative experiment out of three independent experiments are depicted. It can be seen that 60 minutes after induction of cell death, no difference between Nec-1s and primidone can be detected in the efficacy profile. Both compounds still block cell death extremely effectively. In the sample containing primidone, 92.7% of the cells were still alive albeit primidone have been added one hour after induction of cell death. In the sample containing Nec-1s, 85.0% of the cells were still alive in this setting. Primidone, unlike Nec-1s, still protects significantly against cell death even if it was added 3 hours after stimulation of cell death. In the sample containing primidone, 58.6% of the cells were still alive even though primidone was added 3 hour after cell death induction. In the sample containing Nec-1s, only 16.3% of the cells were still in this setting. Therefore, it can be assumed that the longer lasting protective effect exerted by primidone is of enormous importance in everyday clinical practice.

### Example 9: The metabolites PEMA and PB are active as well

Murine fibroblasts (L929 cells) were incubated for 24 h at 37°C in the presence of (a) vehicle, (b) 1 mM PEMA or PB, respectively, (c) a combination of 10 ng/ml TNF-α + 25 µM zVAD (TZ), and (d) a combination of TZ + PEMA or TZ + PB, respectively.

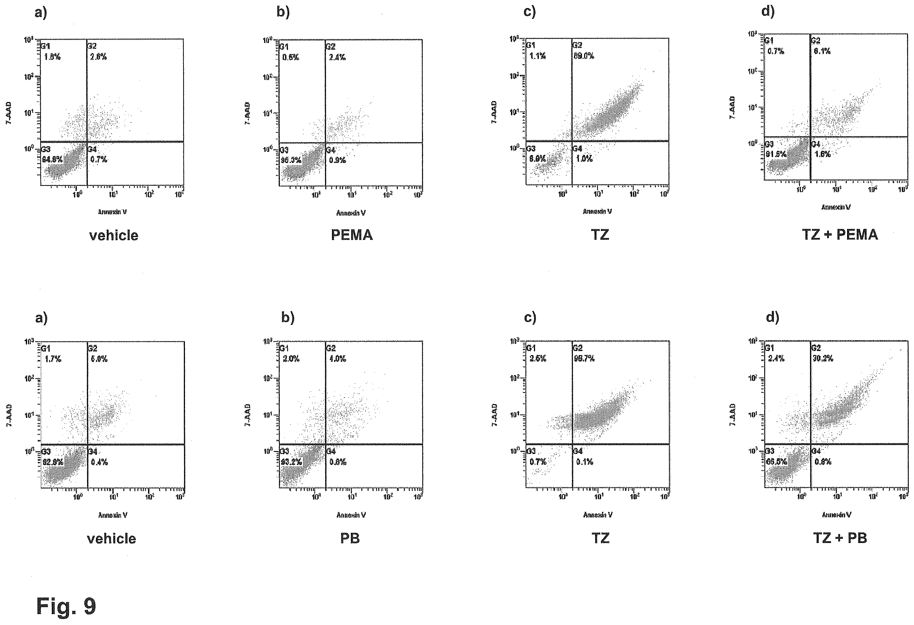

Results: As shown in panels (c) of Figure 9, the combination of TNF-α and zVAD induces programmed cell death. This cell death is, as mentioned before (see Figures 1 and 8), RIPK1-dependent, and it can be blocked both with PEMA and PB, respectively as shown in panels (d). In summary, this experiment shows that the primidone metabolites PEMA and PB can block RIPK1-dependent necroptosis.

### LITERATURE

Cheng, Y. et al. Caspase inhibitor affords neuroprotection with delayed administration in a rat model of neonatal hypoxic-ischemic brain injury. J Clin Invest. 101(9), 1992-1999 (1998).
Cho, Y.S. et al. Phosphorylation-driven assembly of the RIP1-RIP3 complex regulates programmed necrosis and virus-induced inflammation. Cell 137, 1112-1123 (2009).
Degterev A. et al. Targeting RIPK1 for the treatment of human diseases. Proc Natl Acad Sci USA, 116(20), 9714-9722 (2019). Eefing, F. et al. Role of apoptosis in reperfusion injury. Cardiovasc Res. 61(3), 414-426 (2004).
Do YJ eta al. A novel RIPK1 inhibitor that prevents retinal degeneration in a rat glaucoma model. Exp Cell Res, 359(1) :30-38 (2017).
Falcon, C. et al. Exploiting Cell Death Pathways for Inducible Cell Elimination to Modulate Graft-versus-Host-Disease. Bio-medicines, 5(2), 30 (2017).
Galluzzi L. et al. Necroptosis: Mechanisms and Relevance to Disease. Annu Rev Pathol, 12:103-130 (2017).
Gong Y. et al. The role of necroptosis in cancer biology and therapy. Mol Cancer. 18(1), 100 (2019).
Kanou T. et al. Inhibition of regulated necrosis attenuates receptor-interacting protein kinase 1-mediated ischemia-reperfusion injury after lung transplantation. J Heart Lung Transplant, 37(10), 1261-1270 (2018).
Linkermann A. et al. Rip1 (receptor-interacting protein kinase 1) mediates necroptosis and contributes to renal ischemia/reperfusion injury. Kidney Int, 81(8), 751-61 (2012).
Liu C. et al. Necroptosis: A novel manner of cell death, associated with stroke. Int J Mol Med, 41(2), 624-630 (2017)).
Oerlemans MI, et al. Inhibition of RIP1-dependent necrosis prevents adverse cardiac remodeling after myocardial ischemia-reperfusion in vivo. Basic Res Cardiol, 107(4), 270 (2012).
Pavlosky A. et al. RIPK3-mediated necroptosis regulates cardiac allograft rejection. Am J Transplant, 14(8), 1778-90 (2014).
Rickard, J.A. et al. RIPK1 regulates RIPK3-MLKL-driven systemic inflammation and emergency hematopoiesis. Cell 157, 1175-1188 (2014).
Sato K. et al. Receptor interacting protein kinase-mediated necrosis contributes to cone and rod photoreceptor degeneration in the retina lacking interphotoreceptor retinoid-binding protein. J Neurosci, 33(44), 17458-68 (2013).
Shi, S. et al. Necroptotic Cell Death in Liver Transplantation and Underlying Diseases: Mechanisms and Clinical Perspective. Liver Transpl., 25(7), 1091-1104 (2019).
Sun, L. et al. Mixed lineage kinase domain-like protein mediates necrosis signaling downstream of RIP3 kinase. Cell 148, 213-227 (2012).
Takemoto K. et al. Necrostatin-1 protects against reactive oxygen species (ROS)-induced hepatotoxicity in acetaminophen-induced acute liver failure. FEBS Open Bio, 4, 777-87 (2014).
Zhang, T. et al. CaMKII is a RIP3 substrate mediating ischemia- and oxidative stress-induced myocardial necroptosis. Nat Med. 22(2), 175-82 (2016) .
Zhang, S. et al. Necroptosis in neurodegenerative diseases: a potential therapeutic target. Cell Death Dis., 8(6):e2905 (2017).
Yuan, J. et al. Necroptosis and RIPK1-mediated neuroinflammation in CNS diseases. Nat Rev Neurosci, 20(1):19-33, (2019).
Zelic M. et al. RIP kinase 1-dependent endothelial necroptosis underlies systemic inflammatory response syndrome. J Clin Invest, 128(5), 2064-2075 (2018).

## Claims

1. Primidone or a pharmaceutically acceptable active metabolite, derivative, salt or solvate thereof for use in a method of treating a disease that involves a pathologic level of RIPK1-dependent cell death.

2. Primidone or a pharmaceutically acceptable active metabolite, derivative, salt or solvate thereof for use in a method of claim 1, wherein the disease is selected from the group consisting of a reperfusion injury disease, a systemic inflammatory disease, a transplantation-related disease, a neurodegenerative disease, an autoimmune disease, an ophthalmologic disease, a and a RIPK-expressing tumor disease.

3. Primidone or a pharmaceutically acceptable active metabolite, derivative, salt or solvate thereof for use in a method of claim 1, wherein the reperfusion injury disease is selected from myocardial infarction, stroke, acute kidney failure, and acute liver failure.

4. Primidone or a pharmaceutically acceptable active metabolite, derivative, salt or solvate thereof for use in a method of claim 1, wherein the neurodegenerative disease is selected from Alzheimer's disease, Parkinson's disease, Huntington's disease, and amyotrophic lateral sclerosis (ALS), traumatic brain injury, and multiple sclerosis (MS) .

5. Primidone or a pharmaceutically acceptable active metabolite, derivative, salt or solvate thereof for use in a method of claim 1, wherein the autoimmune disease is selected from ulcerative colitis, Crohn's disease, rheumatoid arthritis, autoimmune cardiomyopathy, autoimmune hepatitis, lupus erythematosus, Graves' disease, Guillain-Barre syndrome (GBS), Hashimoto's thyroiditis, idiopathic thrombocytopenic purpura, juvenile idiopathic arthritis, myasthenia gravis, pemphigus vulgaris, psoriasis, Reiter's syndrome, scleroderma, Sjögren's syndrome, vasculitis, vitiligo, and Wegener's granulomatosis.

6. Primidone or a pharmaceutically acceptable active metabolite, derivative, salt or solvate thereof for use in a method of claim 1, wherein the systemic inflammatory disease is selected from sepsis and systemic inflammatory response syndrome (SIRS).

7. Pharmaceutical composition comprising primidone or a pharmaceutically acceptable active metabolite, derivative, salt or solvate thereof for use in a method of treating a disease or condition that involves a pathologic level of RIPK1-dependent cell death.

8. Pharmaceutical composition for use in a method of claim 7, wherein the disease is selected from the group consisting of a reperfusion injury disease, a systemic inflammatory disease, a transplantation-related disease, a neurodegenerative disease, an autoimmune disease, an ophthalmologic disease, a and a RIPK-expressing tumor disease.

9. Pharmaceutical composition for use in a method of claim 7, wherein the reperfusion injury disease is selected from myocardial infarction, stroke, acute kidney failure, and acute liver failure.

10. Pharmaceutical composition for use in a method of claim 7, wherein the neurodegenerative disease is selected from Alzheimer's disease, Parkinson's disease, Huntington's disease, and amyotrophic lateral sclerosis (ALS), traumatic brain injury, and multiple sclerosis (MS).

11. Pharmaceutical composition for use in a method of claim 7, wherein the autoimmune disease is selected from ulcerative colitis, Crohn's disease, rheumatoid arthritis, autoimmune cardiomyopathy, autoimmune hepatitis, lupus erythematosus, Graves' disease, Guillain-Barre syndrome (GBS), Hashi-moto's thyroiditis, idiopathic thrombocytopenic purpura, juvenile idiopathic arthritis, myasthenia gravis, pemphigus vulgaris, psoriasis, Reiter's syndrome, scleroderma, Sjögren's syndrome, vasculitis, vitiligo, and Wegener's granulomatosis.

12. Pharmaceutical composition for use in a method of claim 7, wherein the systemic inflammatory disease is selected from sepsis and systemic inflammatory response syndrome (SIRS).

13. Pharmaceutical composition for use in a method of any of claims 7-12, wherein said composition is formulated for being administered by injection.

14. Pharmaceutical composition for use in a method of any of claims 7-13, wherein said composition comprises at least one additional inhibitor of apoptosis or necroptosis.

15. Pharmaceutical composition for use in a method of any of claims 7-14, wherein primidone or the pharmaceutically acceptable active metabolite, derivative, salt or solvate thereof is administered daily in an amount of comprise 1-50 mg per kilogram body weight of the patient.
